# EUROPEAN PATENT APPLICATION

(11) **EP 1 775 341 A1**
(43) Date of publication of application: **18.04.2007**
(21) Application number: 05753329.1
(22) Date of filing: 27.06.2005
(51) Int. Cl.: C12N 5/06, A61K 35/30, A61K 35/32, A61K 35/48, A61P 17/02, A61P 43/00

(54) **METHOD OF INDUCING THE DIFFERENTIATION OF AMNION-ORIGIN CELLS AND UTILIZATION OF THE SAME**

(30) Priority: 28.06.2004 JP 2004190284
(71) Applicant: National University of Corporation Hiroshima University, Higashihiroshima-shi Hiroshima 739-0046 (JP)
(72) Inventor: KATO, Yukio c/o Hiroshima Univ. Graduate School, Hiroshima 734-8551 (JP); HARA, Tetsuaki c/o Hiroshima Graduate school, hiroshima 734-8551 (JP); SHAO, Jin, Chang c/o Hiroshima University Graduate, hiroshima 734-8551 (JP); SHIMIZU, Masakazu, Kyoto-shi, Kyoto 606-8304 (JP)
(74) Representative: Patentanwaltskanzlei WILHELM & BECK
(86) International application number: PCT/JP2005/011754
(87) International publication number: WO 2006/001428

(57) **Abstract**

By a method of inducing differentiation of cells having: a step of co-culturing amniotic epithelial cells and amniotic interstitial cells so as to induce their differentiation into predetermined tissue cells, it is possible to efficiently induce differentiation of the amniotic epithelial cells and the amniotic interstitial cells. It is preferable that the amniotic epithelial cells or the amniotic interstitial cells are prepared by being cultured by a predetermined culture method.

## Description

### TECHNICAL FIELD

The present invention relates to (a) a differentiation inducing method using a culture method which allows mammalian amnion-derived cells to proliferate while being undifferentiated and (b) utilization of the differentiation inducing method.

### BACKGROUND ART

Attention has been given to regenerative medicine (regenerative medical treatments) as a medical treatment for the twenty first century. A key to the success of regenerative medicine is stem cells.

Known stem cells include embryonic stem cells (ES cells), stem cells of epidermis and sperm, neural stem cells, mesenchymal stem cells, hematopoietic stem cells, and myelogenic stem cells. In particular, the ES cells are enormously useful because of their totipotency to differentiate into any type of cell. However, the ES cells are established from an inner cell mass of the blastocyst in the early embryo of an animal, and the handling of the ES cells has been strictly regulated on bioethical grounds.

In view of this, research regarding stem cells having multipotency (pluripotency) other than the ES cells has been actively conducted. In order to utilize stem cells for regenerative medical treatments of tissues, it is necessary to develop multistep techniques, such as collection of the stem cells from body tissues first, proliferation of the collected stem cells, and further differentiation and proliferation of the proliferated stem cells to prepare a tissue. Among those techniques, the development of a technique of proliferating stem cells in quantities large enough for use as material for regeneration of a tissue, while being undifferentiated, i.e. while retaining their multipotency is essential for the realization of regenerative medical treatments.

As a method of culturing such stem cells, the present inventors have reported that it is possible to obtain a sufficient amount of bone marrow-derived mesenchymal stem cells, for example, by culturing pluripotent mesenchymal stem cells that exist in bone marrow and the like of a mammal and differentiating them into adipocytes, chondrocytes, and osteocytes in the presence of an extracellular matrix and fibroblast growth factor (See Patent documents 1 and 2).

Apart from the mesenchymal stem cells, amnion-derived cells of mammals are known as pluripotent cells, and attempts have been made to utilize the amnion-derived cells for regenerative medicine. For example, Patent document 3 discloses that it is possible to improve clinical conditions of diabetes and bone metabolism disorders by administering human amnion-derived epithelial cells or mesenchymal cells to an organism under certain conditions. Further, Patent document 4 discloses that it is possible to separate and proliferate neural stem cells from a human amnion. Apart from these techniques, many attempts to apply amnion-derived cells to regenerative medicine have been reported as shown in Non-patent document 1.
[Patent document 1]
Japanese Unexamined Patent Publication No. 52360/2003 (Tokukai 2003-52360; published on February 25, 2003)
[Patent document 2]
Japanese Unexamined Patent Publication No. 52365/2003 (Tokukai 2003-52365; published on February 25, 2003)
[Patent document 3]
Japanese Unexamined Patent Publication No. 231639/2003 (Tokukai 2003-231639; published on August 19, 2003)
[Patent document 4]
Japanese Unexamined Patent Publication No. 125759/2003 (Tokukai 2003-125759; published on May 7, 2003)
[Non-patent document 1]

Toshio NIKAIDO, etc., "Application of amnion cells to regenerative medicine", Shinshu Medical Journal, 52(1): 7-14, 2004

As described previously, it has been suggested that mammalian amnion-derived cells are multipotent and able to differentiate into various kinds of cells, and mammalian amnion-derived cells appear to be a highly promising material for regenerative medical treatments. However, a technique of proliferating amnion-derived cells in large quantities while being undifferentiated has never been developed before.

That is, Patent documents 1 and 2 disclose a method of proliferating bone marrow-derived mesenchymal stem cells, which are different from amnion-derived cells in their origins and properties. More specifically, of all the cells constituting an amnion, amnion epithelial cells are derived from epiblast where a fetus is formed, and amnion interstitial (mesenchymal) cells are derived from extraembryonic mesoderm. On the other hand, bone marrow-derived mesenchymal stem cells are derived from epiblast or mesoblast. Thus, it can be said that the bone marrow-derived mesenchymal stem cells are clearly different from the amnion-derived cells in their origins and properties. Because of this, it is uncertain whether the amnion-derived cells and the bone marrow-derived mesenchymal stem cells can be cultured by a similar method. Whether stem cells different in their types, origins and properties can be cultured by the same method must be verified by complicated and advanced experiments beyond the level of trial and error expected of a person skilled in the art. This is obvious from the fact that mouse ES cells lose their differentiation potential in cases where the mouse ES cells are cultured by the method disclosed in Patent documents 1 and 2, as described later.

Further, the technique disclosed in Patent document 3 is a technique of almost directly using amnion-derived cells which have been collected from a living body so as to prepare a medical composition. Patent document 3 fails to disclose or suggest a method for culturing amnion-derived epithelial cells and interstitial cells in large quantities. Patent document 4 discloses a method of selectively culturing only neural stem cells which have been separated from an amniotic mesenchymal cell layer. However, Patent document 4 fails to specifically disclose a culture method for proliferating amnion-derived epithelial cells and interstitial cells in large quantities while being undifferentiated.

In short, although it has been known that the amnion-derived cells are potentially useful as cells for transplant in various regenerative medical treatments because of their capability of differentiating into cells of various tissues such as nerve, a method for proliferating amnion-derived cells in large quantities in vitro to utilize the proliferated cells for regenerative medical treatments was not developed.

Non-patent document 1 has reported that undifferentiated amnion-derived epithelial cells and mesenchymal cells have similarities in properties with undifferentiated ES cells. However, Non-patent document 1 is totally silent about whether their properties can be retained even when the amnion-derived cells are proliferated in large quantities in vitro. In other words, a culturing technique for proliferating amnion-derived epithelial cells and interstitial cells in large quantities while retaining such properties that are similar to properties of undifferentiated ES cells has never been developed.

Under the circumstances, there has been a strong demand for the development of a culture method for proliferating in large quantities in vitro amnion-derived epithelial cells and interstitial cells, which are considered to be enormously useful for regenerative medicine and cell technology, while being undifferentiated.

Furthermore, there has been a strong demand for the development of a technique of efficiently inducing differentiation of amnion-derived epithelial cells and interstitial cells proliferated in large quantities.

### DISCLOSURE OF INVENTION

The present invention has been attained in view of the foregoing problems and an object of the invention is to provide a culture method for proliferating amnion-derived epithelial cells and interstitial cells in large quantities in vitro while being undifferentiated and utilization of the culture method.

The inventors of the present invention diligently worked to solve the foregoing problems and accomplished the present invention by finding that it is possible to proliferate amnion-derived epithelial cells or interstitial cells in large quantities while being undifferentiated by using fibroblast growth factor or a culture dish which is coated with basement membrane extracellular matrix. The present invention has been completed on the basis of such new findings and includes the foregoing inventions:
(1) A method of inducing differentiation of cells having: a step of co-culturing amniotic epithelial cells and amniotic interstitial cells so as to induce their differentiation into predetermined tissue cells;
(2) The method according to (1), wherein: the amniotic epithelial cells and/or the amniotic interstitial cells are mammalian-derived cells which are prepared by a culture method of culturing in the presence of basement membrane extracellular matrix;
(3) The method according to (2), wherein: the culture method is a method of culturing the amniotic epithelial cells or the amniotic interstitial cells on a culture dish coated with basement membrane extracellular matrix;
(4) The method according to (2) or (3), wherein: the basement membrane extracellular matrix is a product formed by culturing PYS2 cells or bovine corneal endothelial cells;
(5) The method according to any one of (2) through (4), wherein: the basement membrane extracellular matrix contains heparin sulfate, dermatan sulfate, type I collagen, type III collagen, type IV collagen, type V collagen, and laminin;
(6) The method according to (1), wherein: the amniotic epithelial cells and/or the amniotic interstitial cells are mammalian-derived cells which are prepared by a culture method of culturing in the presence of fibroblast growth factor;
(7) The method according to any one of (2) through (6), wherein: in the culture method, the amniotic epithelial cells and/or the amniotic interstitial cells proliferate while retaining their multipotency;
(8) The method according to any one of (2) through (7), wherein: in the cells proliferated by the culture method, expression of Oct-4 gene is confirmed by RT-PCR;
(9) The method according to any one of (1) through (8), wherein: the predetermined tissue cells are osteocyte, chondrocyte, or neurocyte;
(10) Cells which can be prepared by the method according to any one of (1) through (9);
(11) The cells according to (10), wherein: said cells are used for regeneration and/or repair of a tissue;
(12) A medical agent used for regeneration and/or repair of an animal tissue, containing the cells according to (10) or (11);
(13) A method for regenerating an animal tissue, wherein: the cells according to (10) or (11) are transplanted alone or with a carrier to a missing part in a tissue and/or a part to be repaired in a tissue; and
(14) A method for manufacturing a medical agent used for regeneration and/or repair of an animal tissue, by using the cells according to (10) or (11).

### BRIEF DESCRIPTION OF DRAWINGS

Fig. 1 is a view showing a result of the promotion of proliferation of amniotic interstitial cells in the presence of FGF.
Fig. 2 is a view showing a result of the promotion of proliferation of amniotic interstitial cells in the presence of basement membrane ECM.
Fig. 3(a) is a view showing a result of 40X-lens observation of a product obtained by induction of chondrogenic differentiation (5 weeks) of proliferated amniotic interstitial cells (third generation).
Fig. 3(b) is a view showing a result of 100X-lens observation of a product obtained by induction of chondrogenic differentiation (5 weeks) of proliferated amniotic interstitial cells (third generation).
Fig. 4 is a view showing a result of the examination of expression of a chondrogenic differentiation genetic marker in proliferated amniotic interstitial cells (third generation).
Fig. 5(a) is a view schematically illustrating a method of co-culturing amniotic interstitial cells and amniotic epithelial cells by Cell culture insert.
Fig. 5(b) is a view showing a result of chondrogenic differentiation induction performed by the method of co-culturing amniotic interstitial cells and amniotic epithelial cells by Cell culture insert.
Fig. 6 is a view showing a result of the examination of Oct-4 gene expression in proliferated human amnion-derived cells.
Fig. 7 is a view showing a result of the promotion of proliferation of amniotic epithelial cells in the presence of basement membrane ECM.
Fig. 8(a) is a view showing a result of immunostaining, which was performed to examine expression of neurofilament 2000 that is a neurodifferentiation marker in amniotic epithelial cells which have been amplified in a test tube, and a result of primary culture.
Fig. 8(b) is a view showing a result of immunostaining, which was performed to examine expression of neurofilament 2000 that is a neurodifferentiation marker in amniotic epithelial cells which have been amplified in a test tube, and a result of fifth generation cells which have been subcultured on basement membrane ECM.
Fig. 9(a) is a view showing a result of immunostaining, which was performed to examine expression of cytokeratin 19 in amniotic epithelial cells which have been amplified in a test tube, and a result of cells just after separation.
Fig. 9(b) is a view showing a result of immunostaining, which was performed to examine expression of cytokeratin 19 in amniotic epithelial cells which have been amplified in a test tube, and a result of fifth generation cells which have been subcultured on a plastic dish.
Fig. 9(c) is a view showing a result of immunostaining, which was performed to examine expression of cytokeratin 19 in amniotic epithelial cells which have been amplified in a test tube, and a result of fifth generation cells which have been subcultured on basement membrane ECM.
Fig. 9(d) is a view showing a result of immunostaining, which was performed to examine expression of cytokeratin 19 in amniotic epithelial cells which have been amplified in a test tube, and a result of staining of amniotic tissue section in vivo.
Fig. 10(a) is a view showing a result of culturing mouse undifferentiated ES cells and a result of culturing the cells on basement membrane ECM, as a reference example of the present invention.
Fig. 10(b) is a view showing a result of culturing mouse undifferentiated ES cells and a result of culturing the cells on a plastic dish, as a reference example of the present invention.
Fig. 10(c) is a view showing a result of culturing mouse undifferentiated ES cells and a result of culturing on feeder cells, as a reference example of the present invention.

### BEST MODE FOR CARRYING OUT THE INVENTION

The present invention has succeeded in separating cells from an epithelium and an interstitium of an amnion, which is medical waste, and then culturing amniotic epithelial cells and amniotic interstitial cells. Conventionally, it was difficult to proliferate these cells. However, the use of fibroblast growth factor or a culture dish which is coated with basement membrane extracellular matrix has made it possible to proliferate cells in large quantities. These cells are capable of differentiating into nerve, bone, cartilage, fat, and others and are therefore a promising material for cells for transplant in regenerative medical treatments. Promising stem cells were (A) ES cells and (B) stem cells existing in, for example, adult's bone marrow, periosteum, and fat tissues. Developmentally, amnion-derived cells are in between the cells (A) and (B). In fact, in the present invention, in view of gene expression, amnion-derived cells which have been amplified in a test tube have similarities in properties with ES cells. Additionally, as with bone marrow-derived mesenchymal stem cells, the amnion-derived cells have osteogenic differentiation potential and chondrogenic differentiation potential. Moreover, unlike ES cells, with which there is no choice but to obtain them from another person, the amnion-derived cells have many advantages in that: if amnion cells derived from a mother are transplanted to her son or daughter or herself, the amnion cells results in less rejection by the recipient; the risk of forming a tumor is low compared with regenerative medical treatments using ES cells; there are less ethical issues as compared with ES cells. For this reason, amnion-derived cells proliferated in large quantity in vitro (e.g. in a test tube) can be expected to serve as cells for regeneration against various kinds of diseases. An embodiment of the present invention that is enormously useful will be described in detail below. It is needless to say that the present invention is not limited to the following descriptions.

### (1) Cell culture method of the present invention

A cell culture method of the present invention is a method of culturing mammalian amnion-derived epithelial cells or interstitial cells in the presence of basement membrane extracellular matrix (basement membrane ECM (Extracellular Matrix)). Other specific conditions (e.g. temperature and carbon dioxide concentration) are not particularly limited and can be set as appropriate.

Mammalian amnion-derived epithelial cells or interstitial cells which are used in the present invention are, literally, ones that are separated and collected from a mammalian amnion. A specific method for the separation and collection is not particularly limited. For example, as shown in Examples described later, it is possible to obtain amniotic epithelial cells and interstitial cells for use in culturing by treating a mammalian amnion by two-step enzymatic decomposition using collagenase and trypsin-EDTA.

Further, a method for obtaining amnion is not particularly limited. For example, in the case of human amnion, amnions can be obtained from placentae which are obtained from patients after childbirth (including natural birth and Caesarean birth) after informed consents are obtained from the patients, as shown in Examples described later. Note that "amniotic epithelial cells" in this specification means (a) cells separated from amniotic epithelium or (b) cells derived from the cells in (a), and "amniotic interstitial cells" in this specification means (i) cells separated from amniotic interstitium or (ii) cells derived from the cells in (i).

A manner of the presence of basement membrane extracellular matrix in a culture medium of the present invention is not particularly limited. However, it is preferable to culture mammalian amnion-derived epithelial cells or interstitial cells on a culture dish coated with basement membrane extracellular matrix. In the culturing, culturing equipment such as a culture dish can be a conventionally known one, and a specific shape, material, type, and the like of the culturing equipment are not particularly limited. It is to be noted that the culture dish coated with basement membrane extracellular matrix can be easily prepared by a person skilled in the art, referring to Reference document (Gospodarowicz D, Cohen, DC, Fujii DK, Regulation of cell growth by the basal lamina and plasma factors, in Cold Spring Harvor Conferences on Cell Proliferation vol 9.G. Sato, A. Pardee and D. Sirbasku eds, 1982; Proc. Natl. Acad. Sci. USA 77, 4094-4098, 1980).

Further, in the present invention, the "basement membrane extracellular matrix" contains at least the following constituents: heparan sulfate, dermatan sulfate, type I collagen, type III collagen, type IV collagen, type V collagen, and laminin. Origins and specific compositions of the constituents are not particularly limited. For example, as shown in the Examples described later, it is preferable to use basement membrane extracellular matrix formed by culturing PYS2 cells or bovine corneal endothelial cells. Apart from PYS2 cells or bovine corneal endothelial cells, a similar basement membrane extracellular matrix can be prepared from, for example, teratocarcinoma-derived PFHR9 cells and other cells.

Furthermore, a cell culture method of the present invention is realized by culturing mammalian amnion-derived epithelial cells or interstitial cells in the presence of fibroblast growth factor (FGF). The "fibroblast growth factor" of the present invention is a family of factors promoting the proliferation of a wide variety of cells which are generated from mesoblast and neuroectoderm. More specifically, examples of the fibroblast growth factor include more than twenty types of molecules such as FGF-1 (acid FGF), FGF-2 (basic FGF), int-2 (FGF-3), hst-1 (FGF-4), FGF-5, hst-2 (FGF-6), KGF (keratinocyte growth factor, FGF-7), FGF-8 (AIGF; androgen-induced growth factor), and FGF-9 (GAF; Glia-activating factor). Among these FGFs, FGF-2 is used in Examples described later. However, this is not the only possibility. The other FGFs can be also preferably used. Concentration and other conditions of FGF can be set in a preferred manner by a person skilled in the art and are not particularly limited. However, it is especially preferable that an FGF concentration is in the range from 0.1 ng/ml to 10 ng/ml.

A medium for use in the culture method of the present invention can be a normal cell culture medium. Specific composition and the like of the medium are not limited. For example, the medium can be MEM (Minimal Essential Medium) or DMEM (Dulbecco's modified Eaqle's medium) mixed with an FBS (Fetal Bovine Serum) and an antibiotic both of which are mixed in a predetermined ratio. If necessary, the medium may be optionally mixed with a growth factor, i.e. a growth contributing substance such as cytokine, platelet concentrate, BMP, TGF-β, IGF, PDGF, VEGF, HGF, and/or combinations of any of these substances. Additionally, the medium may be optionally mixed with a hormone drug such as estrogen, and/or nutriment such as vitamin. The fetal bovine serum may be replaced by human serum. The antibiotic can be any antibiotic such as not only penicillin antibiotic, but also cephem antibiotic, macrolide antibiotic, tetracycline antibiotic, fosfomycin antibiotic, aminoglycoside antibiotic, and newquinolone antibiotic.

Further, in the present invention, an additive being made of any one or combinations of two or more of substances selected from a group consisting of transferrin, insulin, selenic acid, and linoleic acid is mixed into the medium. This makes it possible to cause a proliferation effect which is equal to or more than the effect that is caused by a normal use amount of serum, even when a serum concentration is low in the medium.

As described above, according to the cell culture method of the present invention, it is possible to proliferate mammalian amnion-derived epithelial cells or interstitial cells in large quantities in vitro while retaining their multipotency, i.e. while being undifferentiated.

Moreover, as described later, in amniotic epithelial cells or amniotic interstitial cells which are proliferated by the cell culture method of the present invention, expression of the Oct-4 gene is confirmed by RT-PCR. The Oct-4 gene is a transcription factor that specifically expresses in undifferentiated ES cells, and the Oct-4 gene is considered as being deeply involved in totipotency in differentiation of undifferentiated ES cells. Hence, it is considered to be highly likely that the amniotic epithelial cells or amniotic interstitial cells proliferated by the culture method of the present invention have a property similar to a property of the undifferentiated ES cells, i.e. totipotency in differentiation, and it can be said that the amniotic epithelial cells or amniotic interstitial cells proliferated by the culture method of the present invention are enormously useful for regenerative medical treatments, cell technology, etc. In other words, it can be said that the cell culture method of the present invention is a technique that allows amnion-derived cells to proliferate in large quantities while retaining properties similar to properties of undifferentiated ES cells.

### (2) Cell differentiation inducing Method of the present invention

A cell differentiation inducing method of the present invention is a method of inducing differentiation of the cells prepared by the cell culture method of Section (1) into predetermined tissue cells by using a predetermined differentiation inducing medium. Other specific conditions (e.g. temperature and carbon dioxide concentration) are not particularly limited and can be set as appropriate.

For the induction of differentiation of the proliferated amniotic epithelial cells or amniotic interstitial cells into tissue cells, a preferred differentiation inducing medium can be selected in accordance with their intended purposes. For example, when the "predetermined tissue cells" are osteocyte, chondrocyte, or neurocyte, osteogenic differentiation inducing medium, chondrogenic differentiation inducing medium, or neurodifferentiation inducing medium is used respectively.

As shown in Examples described later, the osteogenic differentiation inducing medium can be, for example, a differentiation inducing medium having a composition including αMEM, FBS, dexamethasone, β-glycerol phosphate, and ascorbic acid-2-posphoric acid. The chondrogenic differentiation inducing medium can be, for example, a differentiation inducing medium having a composition including high-glucose αMEM, TGF-β, dexamethasone, ascorbic acid-2-phosphoric acid, and sodium pyruvate.

As shown in the Examples described later, by co-culturing the amniotic epithelial cells and the amniotic interstitial cells both of which have been prepared by the cell culture method of Section (1), it is possible to further promote induction of differentiation into tissue cells. Especially, it has been proven that induction of osteogenic differentiation is promoted by co-culture of amniotic epithelial cells and amniotic interstitial cells.

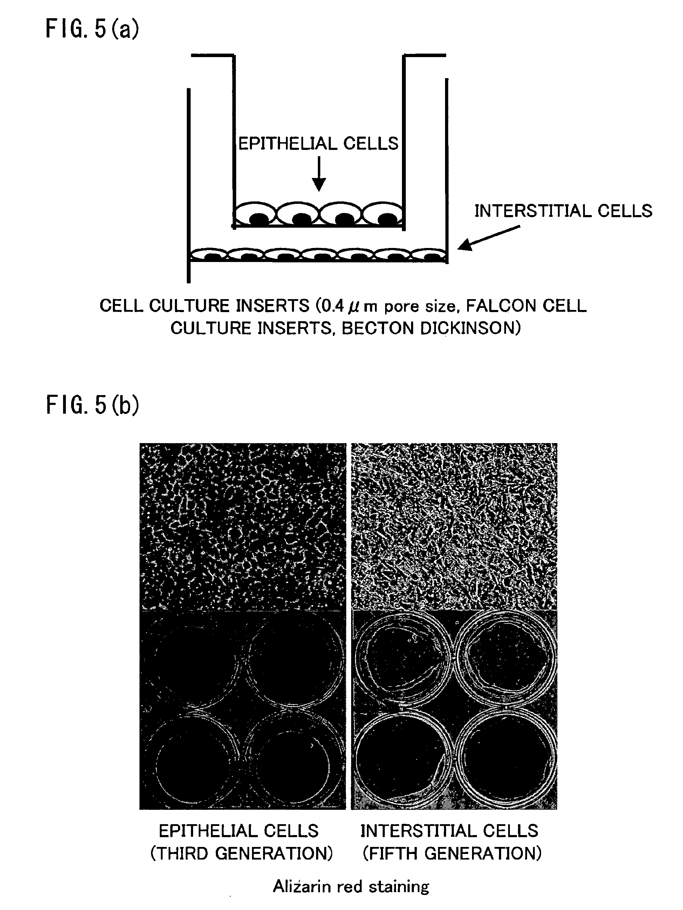

A co-culture method can be a conventionally known method, and can be, but is not particularly limited to, for example, a method using cell culture inserts as shown in Fig. 5(a) of the Examples described later.

As described above, the cells prepared by the cell culture method of Section (1) are considered to have totipotency in differentiation or multipotency in differentiation, which is close to the totipotency in differentiation of ES cells. Hence, differentiation of the cells prepared by the cell culture method of Section (1) into tissue cells is not limited to differentiation into osteocyte, chondrocyte, and neurocyte. It is also possible to differentiate the cells into any other various tissue cells, such as beta cells of the pancreas or hepatocyte (see Non-patent document 1).

### (3) Cells of the present invention and utilization of the cells (medical agent, manufacturing method of the medical agent, and regeneration method)

Cells of the present invention are cells that have been prepared by the cell culture method of Section (1) or cells that can be prepared by the cell culture method of Section (1). In the above cells, expression of the Oct-4 gene is confirmed by RT-PCR. Expression of the Oct-4 gene in the cultured and proliferated amniotic epithelial cells or amniotic interstitial cells has not been reported. The present invention is the first to clarify that the cultured and proliferated amniotic epithelial cells or amniotic interstitial cells have a function like the function of undifferentiated ES cells. Hence, the amniotic epithelial cells or amniotic interstitial cells cultured by the cell culture method of the present invention are considered to have properties similar to the properties of undifferentiated ES cells, i.e. totipotency in differentiation or multipotency in differentiation, which is close to totipotency in differentiation of ES cells. Thus, the amniotic epithelial cells or amniotic interstitial cells cultured by the cell culture method of the present invention are enormously useful in utilization for regeneration and/or repair of animal tissues.

The cells of the present invention are cells that have been prepared by the cell differentiation inducing method of Section (2) or cells that can be prepared by the cell differentiation inducing method of Section (2). As a matter of course, these cells can be also used for regeneration and/or repair of tissues.

That is, the present invention can include a medical agent for regeneration and/or repair of animal tissues, containing the foregoing cells. Similarly, the present invention can include (a) a method for manufacturing a medical agent for regeneration and/or repair of animal tissues by using cells prepared by the foregoing cell culture method and (b) a method for manufacturing a medical agent for regeneration and/or repair of animal tissues by using cells prepared by the foregoing cell differentiation inducing method.

Note that as a matter of course the present invention includes a method of, on the assumption that cells collected from a person are given back to the same person for medical treatment, culturing the collected cells and/or inducing differentiation of the collected cells for the manufacture of a medical agent. More specifically, the cases where the foregoing "medical agent" is used include (A) a case where a manufactured medical agent is used for another person (a person that is not the person who has donated amnion cells) (for example, allotransplantation) and (B) a case where a manufactured medical agent is used for the same person (a person that is the same person who has donated amnion cells) (for example, autotransplantation). Especially, the autotransplantion in which a cell donor and a person who undergoes medical treatment (transplantation) are the same person can be a very excellent regenerative medical treatment since it can almost completely prevent rejection reactions.

The present invention can include an animal tissue regenerating method of transplanting the foregoing cells alone or with a carrier to a missing part of a tissue and/or a part to be repaired in a tissue. The cells of the present invention have a high-level differentiation potential and can be suitably used for regeneration and repair of tissues. The "carrier" used in this specification may be any material provided that it is the one derived from a living organism or a material having affinity to a living tissue, more preferably a bioabsorbable material. Particularly, the carrier may be a porous ceramic having biocompatibility, collagen, polylactic acid, polyglycolic acid, hyaluronic acid, or any combinations of these substances. Alternatively, the carrier may be a metal like titanium. Particularly, in order to regenerate and/or repair a bone, a bone supplement is preferably used. The bone supplement is, for example, a mesoporous material made of a material such as block-formed β-tricalcium phosphate (β-TCP) or hydroxyapatite. Especially, when β-TCP is brought into contact with osteocytes of a missing part of a bone, so-called remodeling, where osteoclasts eat β-TCP and osteoblasts form a new bone, occurs. That is, the bone supplement which has been supplemented to the missing part of the bone is converted into autologous bone with time, and so is enormously useful.

The present invention includes, in addition to the foregoing inventions, the following inventions:
(1) A cell culture method of culturing mammalian amniotic epithelial cells or amniotic interstitial cells in the presence of basement membrane extracellular matrix;
(2) The cell culture method according to (1) wherein: mammalian amniotic epithelial cells or amniotic interstitial cells are cultured on a culture dish coated with the basement membrane extracellular matrix;
(3) The cell culture method according to (1) or (2) wherein: the basement membrane extracellular matrix is formed by culturing PYS2 cells or bovine corneal endothelial cells;
(4) The cell culture method according to any one of (1) through (3) wherein: the basement membrane extracellular matrix contains heparan sulfate, dermatan sulfate, type I collagen, type III collagen, type IV collagen, type V collagen, and laminin.
(5) The cell culture method of culturing mammalian amniotic epithelial cells or amniotic interstitial cells in the presence of fibroblast growth factor.
(6) The cell culture method according to any one of (1) through (5), wherein: the mammalian amniotic epithelial cells or amniotic interstitial cells proliferate while retaining their multipotency.
(7) The cell culture method according to any one of (1) through (6), wherein: in the cells proliferated by the foregoing culture method, expression of the Oct-4 gene is confirmed by RT-PCR.
(8) A cell differentiation inducing method of inducing differentiation of cells prepared by the cell culture method according to any one of (1) through (7) into predetermined tissue cells by using a predetermined differentiation inducing culture.
(9) The cell differentiation inducing method according to (8), wherein: amniotic epithelial cells and amniotic interstitial cells prepared by the cell culture method according to any one of (1) through (7) are co-cultured.
(10) The cell differentiation inducing method according to (8) or (9), wherein: the predetermined tissue cells are osteocyte, chondrocyte, or neurocyte.
(11) Cells which can be prepared by the cell culture method according to any one of (1) through (7).
(12) The cells according to (11), wherein: said cells have multipotency.
(13) Cells which can be prepared by the cell differentiation inducing method according to any one of (8) through (10).
(14) The cells according to any one of (11) through (13), wherein: said cells are used for regeneration and/or repair of a tissue.
(15) A medical agent used for regeneration and/or repair of an animal tissue, containing the cells according to any one of (11) through (14).
(16) A method for regenerating an animal tissue, wherein: the cells according to any one of (11) through (14) are transplanted alone or with a carrier to a missing part of a tissue and/or a part to be repaired in a tissue.
(17) A method for manufacturing a medical agent used for regeneration and/or repair of an animal tissue, by using cells prepared by the cell culture method according to any one of (1) through (7).
(18) A method for manufacturing a medical agent used for regeneration and/or repair of an animal tissue, by using cells prepared by the cell differentiation inducing method according to any one of (8) through (10).

The following will describe Examples to explain details of embodiments of the present invention. The present invention is not limited to the description of the following Examples. It is needless to say that particulars thereof can be in various modes. Further, the present invention is not limited to the description of the foregoing embodiments, and can be altered in many variations, provided such variations do not exceed the scope of the patent claims set forth below. An embodiment based on a proper combination of technical means disclosed is encompassed in the technical scope of the present invention.

### [Examples]

### <1> Separation of amnion cells and proliferation promotion using FGF

### (Cell separation and culture method)

Using placenta which were obtained from women after childbirth (including natural birth and Caesarean birth), from whom informed consent was obtained during pregnancy, amnion was peeled off with tweezers and separated from a chorionic layer. Separation of amnion cells was carried out by two-step enzymatic decomposition using collagenase and trypsin-EDTA. More specifically, the amnion thus obtained was put in a 100 ml beaker and washed with a phosphate buffer (PBS) three times. Thereafter, the amnion was cut into small pieces with sterilized surgical scissors. The pieces of amnion thus obtained were shaken for an hour at 37 °C in a DMEM-F12 (Sigma) with rotation at 500 to 600 revolutions per minute by a stirrer bar in a medium containing 1 mg/ml of collagenase (Sigma) and 0.08 % DNAase (Sigma).

In order to remove amniotic epithelial cells, the resultant solution was passed through a 100µm-filter net and a 40µm-filter net, and collected by centrifuging at 2000xg for 10 minutes to separate amniotic mesenchymal (interstitial) cells. The cells thus obtained were counted by a Coulter counter (Z1 single; manufactured by Coulter, Inc.). By this method, 1.0 × 10⁶ to 2 × 10⁶ interstitial cells were normally collected from 1g of amniotic tissue. The cells thus obtained were inoculated at a density of 4 × 10⁴ cells/cm² upon a 10cm-diameter tissue culture dish in a DMEM-F12 medium (100 units/ml of penicillin and 100 µg/ml of streptomycete) containing 10 % fetal bovine serum (FBS). A primary culture of the cells was carried out at 37 °C in the presence of 5% carbon dioxide.

Amniotic epithelial cells were separated from the amnion from which the mesenchymal cells had been completely removed. The residual amnion on the filter nets was treated with 0.05 % trypsin and 0.2 mM of EDTA at 37 °C for 5 minutes and collected by centrifugation to separate epithelial cells. By this method, approximately 8.0 × 10⁶ to 10 × 10⁶ epithelial cells were normally collected from 1 g of amniotic tissue. The epithelial cells thus obtained were inoculated at a density of 5 × 10⁴ cells/cm² upon a 10cm-diameter tissue culture dish and cultured at 37 °C in the presence of 5% carbon dioxide in a DMEM-F12 medium (100 units/ml of penicillin and 100 µg/ml of streptomycete) containing 10 % FBS.

### (Proliferation promoting method using FGF)

At the time when the primary cultured mesenchymal cells became nearly confluent after about 7 days, the culture dish was treated with 0.05 % trypsin and 0.2 mM of EDTA. Then, the mesenchymal cells were collected from the dish, and the number of the cells thus obtained was counted. The cultured mesenchymal cells were inoculated at a density of 5000 cells/cm² in a DMEM-F12 medium (10% FBS, 100 units/ml of penicillin, and 100 µg/ml of streptomycete) containing or not containing fibroblast growth factor (FGF) -2 (3 ng/ml). On the third day of the culture, the medium was replaced with a new one. Thereafter, the medium was replaced with a new one every three days. Note that FGF-2 (3ng/ml) (FGF-2 is desirably in the range from 0.1 ng/ ml to 10 ng/ml, but amounts of FGF-2 below and above the range is also effective.) was added from the first day of the culture. The cultured cells were subcultured before they became confluent. This operation was carried out again and again for subculturing. Apart from FGF-2, other FGF family members may be added.

### (Result)

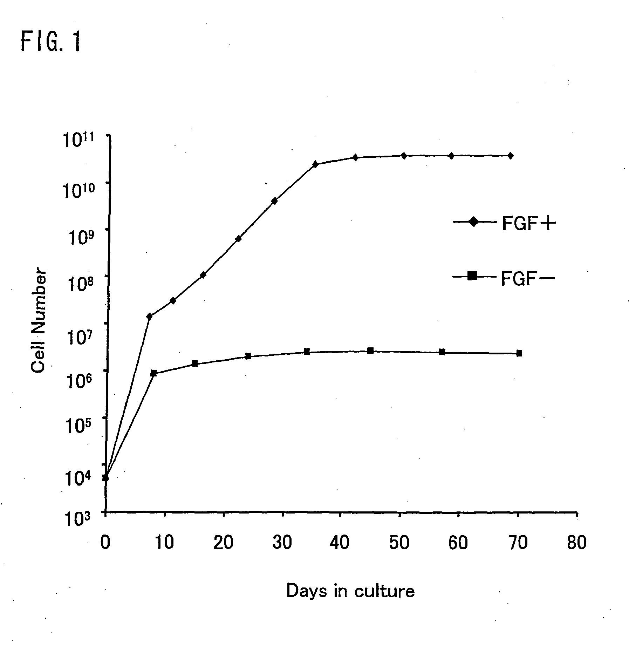

While FGF-2 (3ng/ml) was added to the DMEM-F12 medium containing 10 % FBS, amnion-derived mesenchymal cells had been proliferated and cultured for two months. The result of the proliferation and culturing is shown in Fig. 1. The amnion-derived mesenchymal cells were proliferated to 10⁶ times, or more, of the original cells. Such a high proliferation potency was retained up to the seventh generation (see "FGF+" in Fig. 1). On the other hand, in the conventional method (which does not include FGF), the proliferation stopped after the first subculturing (10 days or more after the culturing) (see "FGF-" in Fig. 1).

### <2> Preparation of a culture dish coated with basement membrane ECM and proliferation of amnion cells on ECM

### (Preparation of culture dish coated with extracellular matrix)

Basement membrane extracellular matrix (ECM) was prepared by using PYS2 cells in accordance with the document (Gospodarowicz D, Cohen, DC, Fujii DK, Regulation of cell growth by the basal lamina and plasma factors, in Cold Spring Harvor Conferences on Cell Proliferation vol 9.G. Sato, A. Pardee and D. Sirbasku eds, 1982; Proc. Natl. Acad. Sci. USA 77, 4094-4098, 1980).

Specifically, the basement membrane extracellular matrix is prepared as follows: First of all, PYS2 cells were cultured until they became confluent. Thereafter, the PYS2 cells were further cultured for a week in the presence of 5 % dextran (Molecular weight: approximately two hundred thousand, Wako, Osaka, Japan), 50 µg/ml of ascorbic acid-2-phosphoric acid (Sigma), and 10 % FBS. To layers of the cells, a dilute ammonia solution (20 mM to 50 mM) was added. After only the cellular components were removed, extracellular matrix which was bonded to and remains on a lower layer of the culture dish was washed with PBS several times and then stored at 4 °C.

### (Culturing of amniotic interstitial cells on basement membrane extracellular matrix)

Third to sixth generation amnion-derived interstitial cells which have been cultured in the FGF-added medium were collected and inoculated on a culture dish coated with the extracellular matrix and on a normal plastic tissue culture dish. The amnion-derived interstitial cells were cultured under the conditions as in the primary culture. As a result, it was observed that the cells that had been cultured by the FGF-added culturing had a spindle-like shape and the cells that had been cultured on the extracellular matrix coated culture dish had a shape longer than the cells that had been cultured by the FGF-added culturing, whereas the cells that had been cultured on the plastic became flattened.

### (Proliferation of amniotic interstitial cells on extracellular matrix)

On plastic dishes for tissue culture use having various diameters, extracellular matrices were prepared according to the foregoing extracellular matrix preparation method, and the amniotic interstitial cells that had been obtained by the foregoing primary culture method were inoculated on the dishes at a density of 5000 cells/cm². After the inoculation, a DMEM medium containing 10 % fetal bovine serum, 100 units/ml of penicillin, and 100 µg/ml of streptomycete was replaced with a new one every three days. Before becoming confluent, the cells on the extracellular matrix were peeled off by 0.2 mM of EDTA containing 0.05 % trypsin. After the obtained cells were centrifuged at 500 × g, a mass of cells precipitated was returned to the DMEM medium for another suspension, and the cells were inoculated on the extracellular matrix again at a density of 5000 cells/cm². This subculturing operation was repeated as long as cell division occurred.

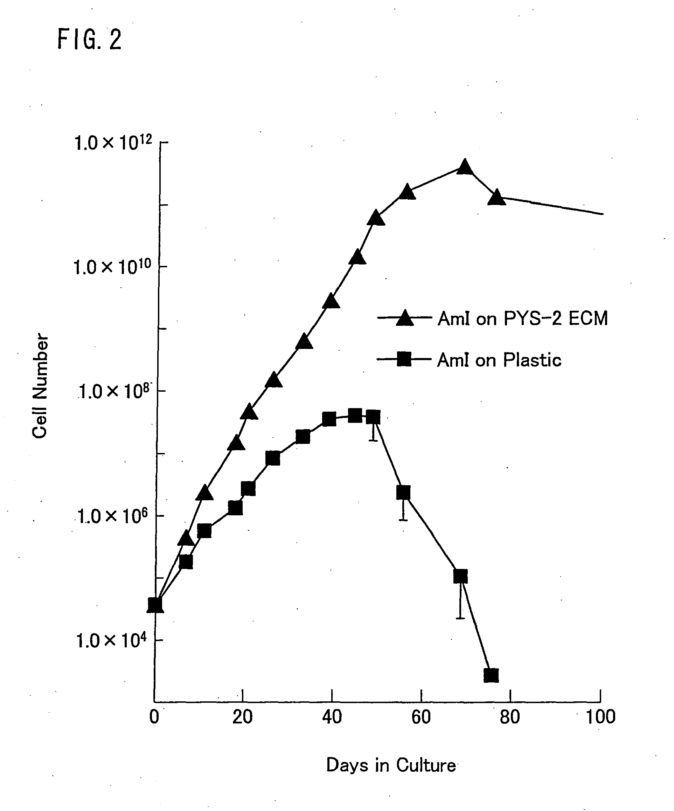

A result of this operation is shown in Fig. 2. As show in Fig. 2, the amniotic interstitial cells that have been cultured on the extracellular matrix were amplified 10⁶ times, or more, of the original cells. This proliferation potency was maintained up to the eleventh generation. In addition, as compared with the conventional method, each lifetime of the cells got longer.

### <3> Induction of differentiation of amnion cells into chondrocyte

### (Induction of differentiation of amnion cells into chondrocyte)

In order to perform induction of differentiation into chondrocyte, third to sixth generation amniotic mesenchymal cells that had been cultured in a FGF-2 added medium were collected and moved to a chondrogenic differentiation inducing medium of a composition shown in Table 1. Two hundred thousand cells were put into a 15 ml centrifuging tube (Falcon) and incubated in 0.5 ml to 1.0 ml of medium.

**[Table 1]**

| Chondrogenic differentiation inducing medium | |
|---|---|
| High glucose αMEM | |
| 10 ng/ml of TGF-β3 | |
| 100 nM of dexamethasone | |
| 50 µg/ml of ascorbic acid-2-phosphoric acid | |
| 100 µg/ml of sodium pyruvate | |
| ITS-plus | 6.25 µg/ml transferrin |
| | 6.25 µg/ml insulin |
| | 6.25 ng/ml selenic acid |
| | 5.33 µg/ml linoleic acid |
| | 1.25 mg/ml of bovine plasma albumin |

In the above medium, amniotic interstitial cells were cultured at 37 °C in the presence of 5 % carbon dioxide. 24 hours after the start of culturing, the cells formed spherical pellets. The cells were cultured for 35 days while the medium was replaced with a new one every three days. As controls, human iliac bone marrow-derived mesenchymal stem cells and human skin fibroblasts were cultured under similar culturing conditions to the amniotic interstitial cells.

A total RNA of the cultured cells was extracted by TRIzol (Life Technologies Inc.). Further, expressions of genetic markers specific to chondrocyte, i.e. Type II collagen and Type X collagen were examined by RT-RCR. An RT-PCR kit, ReverTra Dash (Toyobo Co., Ltd, Osaka, Japan) was used in accordance with protocols attached to the RT-PCR kit. The conditions of PCR were 35 cycles of 30 seconds at 94 °C and 3 minutes at 68 °C. Primers shown below were used. PCR product analysis was carried out by electrophoretic migration.
- Human type II collagen : forward 5'-TGGTGGAGCAGCAAGAGCAA-3' (SEQ ID NO: 1)
- Human type II collagen : reverse 5'-TGCCCAGTTCAGGTCTCTTA-3' (SEQ ID NO: 2)
- Human type X collagen : forward 5'-CCCAACACCAAGACACAGTT-3' (SEQ ID NO: 3)
- Human type X collagen : reverse 5'-CATCACCTTTGATGCCTGGCT-3' (SEQ ID NO: 4)
- Human GAPDH: forward 5'-GTCAAGGCCGAGAATGGGAA-3' (SEQ ID NO: 5)
- Human GAPDH: reverse 5'-GCTTCACCACCTTCTTGATG-3' (SEQ ID NO: 6)

### (Result)

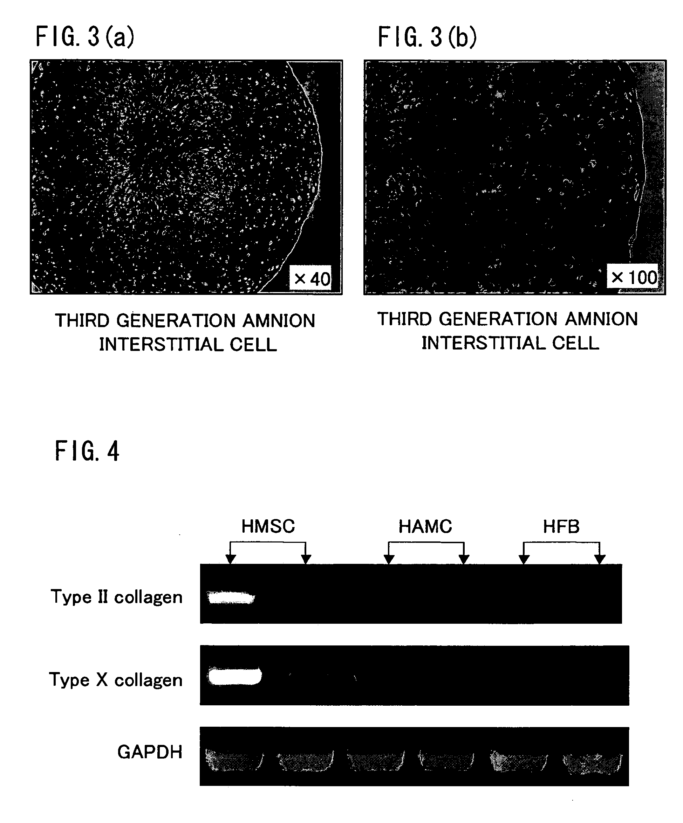

A preparation of the cultured pellet was prepared for toluidine blue staining. A result of the toluidine blue staining is shown in Fig. 3. Fig. 3(a) shows an image observed by a 40X lens. Fig. 3(b) shows an image observed by a 100X lens. As shown in Fig. 3, it was confirmed that the amniotic interstitial cells that had been subjected to induction of chondrogenic differentiation turned into chondrocytes, and that a chondrocyte-specific intercellular material (metachromasia by toluidine blue) was deposited around the chondrocytes due to the toluidine blue staining.

Further, Fig. 4 shows a result of the examination of a chondrocyte-specific genetic marker. As shown in Fig. 4, expressions of Type II collagen gene and Type X collagen gene were weaker in the pellet of amniotic interstitial cells (HAMC; human amniotic mesenchymal cells) than in iliac mesenchymal stem cells (HMSC; human bone marrow derived mesenchymal stem cells), but stronger than in the fibroblast (HFB; human fibroblast).

### <4> Amnion cells that have been amplified in a test tube retain osteogenic differentiation potential.

In order to prove that the amnion cells that have been amplified in a test tube retain osteogenic differentiation potential, amniotic interstitial cells and epithelial cells were co-cultured. For the induction of osteogenic differentiation of these cells, the third to sixth generations of amniotic interstitial cells that had been cultured in the FGF-added medium and the third generation of amniotic epithelial cells that had been cultured in the DMEM-F12 medium were collected, inoculated on cell culture inserts (0.4 µm pore size, Falcon Cell Culture Inserts, Becton Dickinson), and moved to an osteogenic differentiation inducing medium of a composition shown in Table 2 given below.

**[Table 2]**

| Osteogenic differentiation inducing medium |
|---|
| αMEM |
| 10% FBS |
| 100 nM of dexamethasone |
| 10 mM of β-glycerol phosphate |
| 50 µg/ml of ascorbic acid-2-phosphoric acid |

The amniotic interstitial cells and epithelial cells were co-cultured at 37 °C in the presence of 5 % carbon dioxide in the differentiation inducing medium for 28 days while the medium was replaced with a new one every three days. Fig. 5(a) is a view schematically illustrating a co-culture method.

### (Result)

A result of the co-culturing is shown in Fig. 5(b). In Fig. 5(b), upper panels show results of the observation of cells under a microscope, and lower panels show results of the observation of the degree of calcification using the naked eye. As shown in Fig. 5(b), in both the amniotic interstitial cells and epithelial cells that have been co-cultured on the cell culture inserts, calcification that is unique to osteoblast was confirmed by the observations under a microscope. In the observation using the naked eye, alizarine red staining that is indicative of calcification was also confirmed.

### <5> Oct-4 expression in undifferentiated human amnion-derived cells

### (Oct-4 expression in undifferentiated human amnion-derived cells)

From undifferentiated human interstitial cells and epithelial cells which had been cultured under the same conditions as the primary culture, a total RNA was extracted by TRIzol (Life Technologies Inc.). Expression of a genetic marker specific to undifferentiated ES cells, i.e. Human OCT-4 was examined by RT-RCR. An RT-PCR kit, ReverTra Dash (Toyobo Co., Ltd, Osaka, Japan) was used in accordance with protocols attached to the RT-PCR kit. The conditions of PCR were 35 cycles of 1 minute at 94 °C, 1 minute at 60 °C, and 2 minutes at 72 °C. PCR product analysis was carried out by electrophoretic migration. Primers shown below were used.
- Human OCT-4: forward 5'-GAAGCTGGAGAAGGAGAAGCTG-3' (SEQ ID NO: 7)
- Human OCT-4: reverse 5'-CAAGGGCCGCAGCTTACACACATGTTC-3' (SEQ ID NO: 8)
- Human GAPDH: forward 5'-GTCAAGGCCGAGAATGGGAA-3' (SEQ ID NO: 9)
- Human GAPDH: reverse 5'-GCTTCACCACCTTCTTGATG-3' (SEQ ID NO: 10)

### (Result)

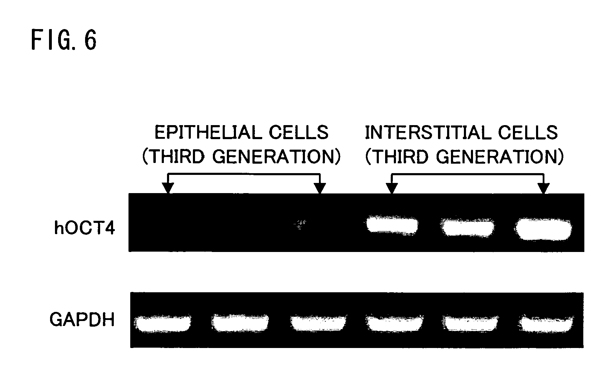

A result of the examination is shown in Fig. 6. Oct-4 specifically expresses in ES cells and is a transcription factor which is essential to their totipotency in differentiation. As a result of the examination of Oct-4 expression in undifferentiated human amnion-derived cells that have been cultured by a culture method of the present invention, Oct-4 expression was confirmed in both the amniotic interstitial cells and the amniotic epithelial cells.

### <6> Promotion of proliferation of amniotic epithelial cells by extracellular matrix culture method and retention of neurodifferentiation potential

On plastic dishes for tissue culture use having various diameters, extracellular matrix was prepared according to the foregoing extracellular matrix preparation method, and the amniotic interstitial cells that had been obtained by the foregoing primary culture method were inoculated on the dishes at a density of 50000 cells/cm². After the inoculation, a DMEM/F12 medium containing 10 % fetal bovine serum, 100 units/ml of penicillin, and 100 µg/ml of streptomycete was replaced with a new one every three days. Before becoming confluent, the cells on the extracellular matrix were peeled off by 0.2 mM of EDTA containing 0.05 % trypsin. After the obtained cells were centrifuged at 500g, a mass of precipitated cells was returned to the DMEM/F12 medium for another suspension, and the cells were inoculated on the extracellular matrix again at a density of 50000 cells/cm². This subculturing operation was repeated.

Thereafter, in order to confirm whether the amniotic epithelial cells that had been cultured by the foregoing extracellular matrix method retained their neurodifferentiation potential even after they have been proliferated, immunostaining with Neuro Filament 200 (Sigma), which is a neurodifferentiation marker, was performed.

In addition, immunostaining with cytokeratin 19, which is an epithelial cell marker, was performed to confirm whether properties of epithelial cells were retained.

### (Result)

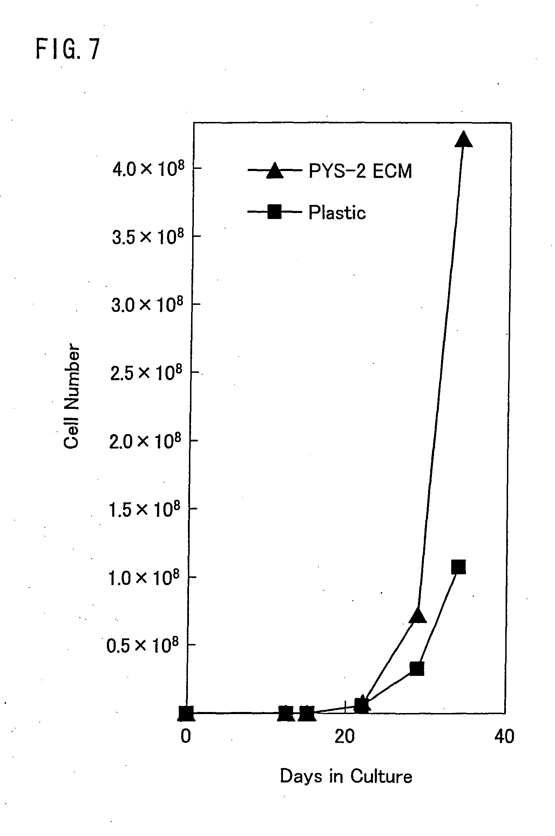

First, a result of the culturing of cells is shown in Fig. 7. As shown in Fig. 7, the amniotic epithelial cells that have been cultured on the extracellular matrix were proliferated 1000 times, or more, of their original cells. This proliferation potency is superior to that obtained by the conventional method, and the proliferation potency was retained to the fifth or more generations.

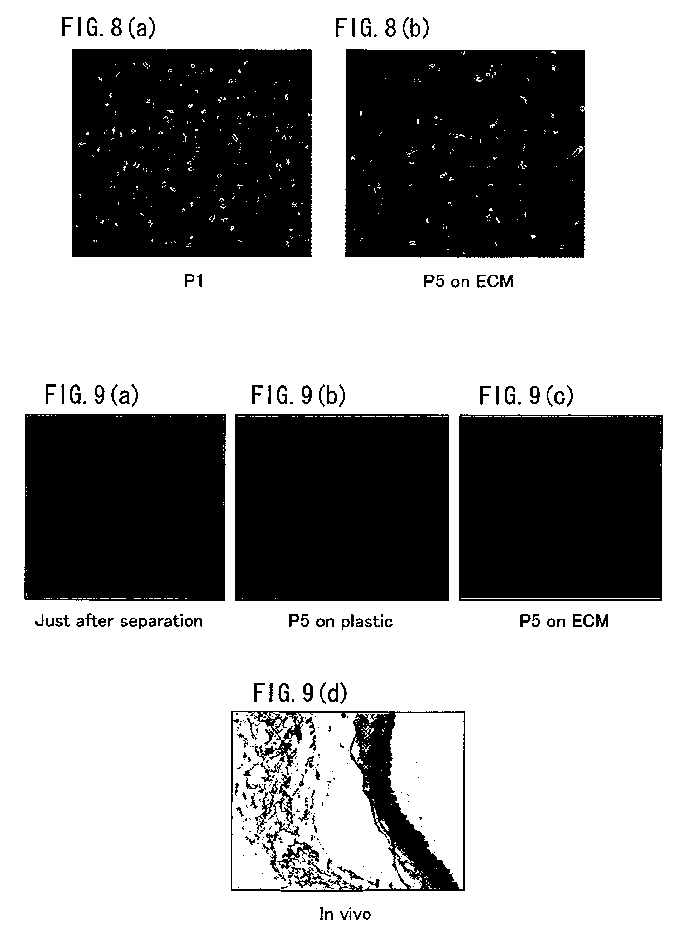

Next, a result of the detection of the neurodifferentiation marker is shown in Fig. 8. Fig. 8(a) shows a result of staining of primary cultured cells (P1). Fig. 8(b) shows a result of staining of fifth generation cells (P5). As shown in Fig. 8, as a result of immunostaining of the proliferated cells with the neurodifferentiation marker, the cells proliferated 1000 times or more were stained as with the primary cultured cells. This confirmed that differentiation potential was retained.

In addition, a result of immunostaining with cytokeratin19 is shown in Fig. 9. Fig. 9(a) shows a result of staining of cells just after separation. Fig. 9(b) shows a result of staining of fifth generation cells (P5) which have been cultured on a plastic dish for tissue culture use. Fig. 9(c) is a result of staining of fifth generation cells (P5) which have been cultured on basement membrane extracellular matrix. Fig. 9(d) shows a result of staining of cells in vivo. As shown in these drawings, from the result of immunostaining with cytokeratin 19, the cells were confirmed to be epithelial cells. The above results suggest that the increase of amnion cells, to be used for regenerative medical treatments in large quantities, allows for the utilization of the amnion cells with their usefulness as a cell supply source further increased. <Reference example> Culturing of mouse ES cells on basement membrane extracellular matrix or in the presence of FGF

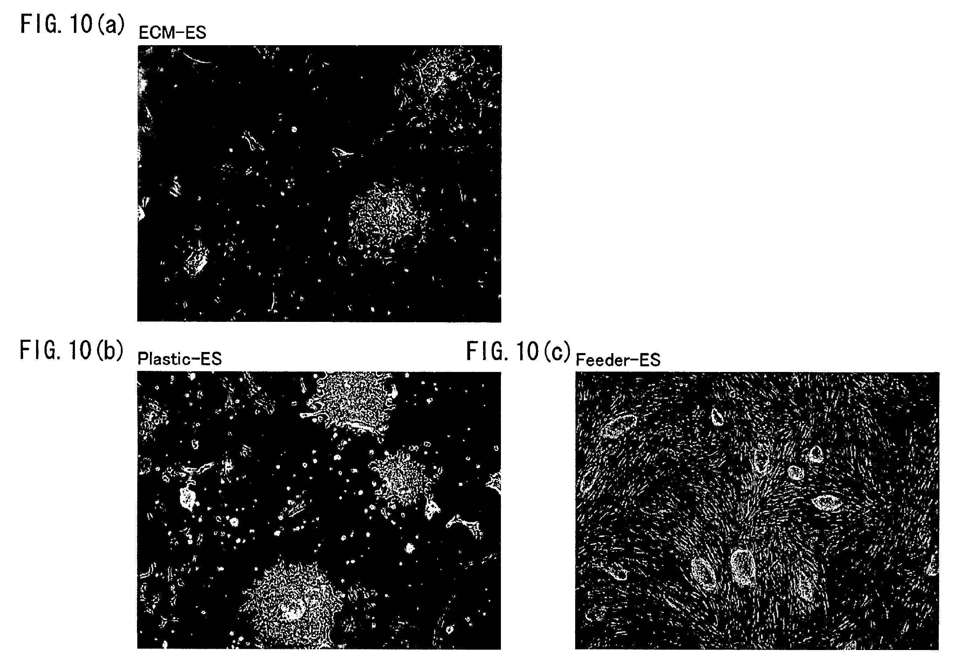

Whether mouse ES cells can be cultured on basement membrane extracellular matrix or in the presence of FGF was examined. More specifically, on plastic dishes for tissue culture use having various diameters, extracellular matrices were prepared according to the foregoing extracellular matrix preparation method, and mouse ES cells were cultured on the dishes. A result of the examination is shown in Figs. 10(a) through 10(c).

Fig. 10(a) shows a result of culturing mouse ES cells on the extracellular matrix. Fig. 10(b) shows a result of culturing mouse ES cells on a plastic dish. Fig. 10(c) shows a result of culturing mouse ES cells on feeder cells as a positive control. As shown in Figs. 10(a) through 10(c), in the case where mouse ES cells were cultured on feeder cells, mouse ES cells were proliferated, as a undifferentiated colony, with their cell boundaries unclearly defined. In the case were mouse ES cells were cultured on the extracellular matrix, and in the case where ES cells were cultured on the plastic dish, the mouse ES cells differentiated and changed to cobblestone-like cells. In addition, the mouse ES cells were unable to remain undifferentiated in the presence of FGF, although this result is not shown. From these results, it was found that in the case where mouse ES cells were cultured on the basement membrane extracellular matrix or in the presence of FGF, it was difficult to culture the mouse ES cells while they remained undifferentiated.

### INDUSTRIAL APPLICABILITY

A cell culture method of the present invention has the effect of proliferating mammalian amnion-derived epithelial cells or interstitial cells, which are useful for regenerative medical treatments and cell technology, in large quantities while they remain undifferentiated. Further, by using a differentiation inducing method of the present invention, it is possible to easily and reliably differentiate mammalian amnion-derived epithelial cells or interstitial cells into target tissue cells.

Still further, by using cells which have been prepared by the foregoing culture method or the differentiation inducing method, it is possible for the cells to be applied to medical products for regeneration and/or repair of animal tissue and regeneration methods.

An amnion has a thick basement membrane made of collagen (Type III, IV, V), laminin, and nidgen is present in the amnion, has characteristics which other tissues do not have, and has a nature in which rejection reactions are unlikely to occur because it does not contain blood vessels. This allows regenerative medical treatments using the amnion-derived cells to have the advantage of reduced rejection reactions.

Further, since the amnion is medical waste, there are less ethical issues and it is relatively easier to obtain primary cells for culturing, as compared with ES cells.

As described above, a cell culture method and its utilization of the present invention is applicable to a wide range of industries including biotechnology, regenerative medical treatments, cell technology and other technologies. More specifically, cells of the present invention can be used for artificially cultured bone, artificially cultured cartilage, osteogenic differentiation accelerator, and cells for repair of various tissues, for example.

## Claims

1. A method of inducing differentiation of cells comprising:
the step of co-culturing amniotic epithelial cells and amniotic interstitial cells to induce their differentiation into predetermined tissue cells.

2. The method according to claim 1, wherein:
the amniotic epithelial cells and/or the amniotic interstitial cells are mammalian-derived cells which are prepared by a culture method in which culturing is carried out in the presence of basement membrane extracellular matrix.

3. The method according to claim 2, wherein:
the culture method is a method of culturing amniotic epithelial cells or amniotic interstitial cells on a culture dish coated with basement membrane extracellular matrix.

4. The method according to claim 2 or 3, wherein:
the basement membrane extracellular matrix is a product formed by culturing PYS2 cells or bovine corneal endothelial cells.

5. The method according to any one of claims 2 through 4, wherein:
the basement membrane extracellular matrix contains heparin sulfate, dermatan sulfate, type I collagen, type III collagen, type IV collagen, type V collagen, and laminin.

6. The method according to claim 1, wherein:
the amniotic epithelial cells and/or the amniotic interstitial cells are mammalian-derived cells which are prepared by a culture method in which culturing is carried out in the presence of fibroblast growth factor.

7. The method according to any one of claims 2 through 6, wherein:
in the culture method, the amniotic epithelial cells and/or the amniotic interstitial cells proliferate while retaining their multipotency.

8. The method according to any one of claims 2 through 7, wherein:
in the cells proliferated by the culture method, expression of Oct-4 gene is confirmed by RT-PCR.

9. The method according to any one of claims 1 through 8, wherein:
the predetermined tissue cells are osteocyte, chondrocyte, or neurocyte.

10. Cells which can be prepared by a method recited in any one of claims 1 through 9.

11. The cells according to claim 10, wherein:
said cells are used for regeneration and/or repair of a tissue.

12. A medical agent used for regeneration and/or repair of an animal tissue, containing cells recited in claim 10 or 11.

13. A method for regenerating an animal tissue, wherein:
cells recited in claim 10 or 11 are transplanted alone or with a carrier to a missing part in a tissue and/or a part to be repaired in a tissue.

14. A method for manufacturing a medical agent used for regeneration and/or repair of an animal tissue, by using cells recited in claim 10 or 11.


### [Sequence List]

Sequence List (HU0515_PCT).app

SEQUENCE LISTING

<110> National University of Corporation Hiroshima University

<120> Methods for culturing of amniotic cells and use
      thereof

<130> HU0515

<150> JP 2004-190284
<151> 2004-6-28

<160> 10

<170> PatentIn Ver. 2.1

<210> 1
<211> 20
<212> DNA
<213> Artificial Sequence

<220>
<223> Description of Artificial Sequence: Human type II
      collagen: forward primer

<400> 1
tggtggagca gcaagagcaa                                              20

<210> 2
<211> 20
<212> DNA
<213> Artificial Sequence

<220>
<223> Description of Artificial Sequence: Human type II

collagen: reverse primer

<400> 2
tgcccagttc aggtctctta                                                    20


<210> 3
<211> 20
<212> DNA
<213> Artificial Sequence

<220>
<223> Description of Artificial Sequence: Human type X
      collagen: forward primer

<400> 3
cccaacacca agacacagtt                                                    20


<210> 4
<211> 21
<212> DNA
<213> Artificial Sequence

<220>
<223> Description of Artificial Sequence: Human type X
      collagen: reverse primer

<400> 4
catcaccttt gatgcctggc t                                                  21


<210> 5
<211> 20
<212> DNA
<213> Artificial Sequence

<220>
<223> Description of Artificial Sequence: Human GAPDH:
      forward primer

<400> 5
gtcaaggccg agaatgggaa                                                    20


<210> 6
<211> 20
<212> DNA
<213> Artificial Sequence

<220>
<223> Description of Artificial Sequence: Human GAPDH:
      reverse primer

<400> 6
gcttcaccac cttcttgatg                                                    20


<210> 7
<211> 22
<212> DNA
<213> Artificial Sequence

<220>
<223> Description of Artificial Sequence: Human OCT-4:
      forward primer

<400> 7
gaagctggag aaggagaagc tg                                                 22


<210> 8

<211> 27
<212> DNA
<213> Artificial Sequence

<220>
<223> Description of Artificial Sequence: Human OCT-4:
      reverse primer

<400> 8
caagggccgc agcttacaca catgttc                                          27


<210> 9
<211> 20
<212> DNA
<213> Artificial Sequence

<220>
<223> Description of Artificial Sequence: Human GAPDH:
      forward primer

<400> 9
gtcaaggccg agaatgggaa                                                  20


<210> 10
<211> 20
<212> DNA
<213> Artificial Sequence

<220>
<223> Description of Artificial Sequence: Human GAPDH:
      reverse primer

<400> 10
gcttcaccac cttcttgatg                                                  20